# EUROPEAN PATENT SPECIFICATION

(11) **EP 4 041 186 B1**
(45) Date of publication and mention of the grant of the patent: **22.05.2024**
(21) Application number: 20789063.3
(22) Date of filing: 07.10.2020
(51) Int. Cl.: A61P 17/16, A61K 9/00, A61K 9/12, A61K 31/18, A61K 31/573, A61K 31/593

(54) **AN ANHYDROUS PHARMACEUTICAL COMPOSITION FOR MAINTENANCE TREATMENT OF PSORIASIS**
WASSERFREIE PHARMAZEUTISCHE ZUSAMMENSETZUNG ZUR ERHALTUNGSTHERAPIE VON PSORIASIS
COMPOSITION PHARMACEUTIQUE ANHYDRE POUR LE TRAITEMENT DE MAINTENANCE DU PSORIASIS

(30) Priority: 08.10.2019 GB 201914498
(43) Date of publication of application: 17.08.2022
(73) Proprietor: LEO Pharma A/S, 2750 Ballerup (DK)
(72) Inventor: BANG, Bo, 2750 Ballerup (DK); HANSEN, Klaus Krog, 2750 Ballerup (DK); LILJEDAHL, Monika, 2750 Ballerup (DK)
(74) Representative: Zacco Denmark A/S
(86) International application number: PCT/EP2020/078126
(87) International publication number: WO 2021/069496

(56) References cited:
- WO-A1-2011/154004
- J.-H. LEE ET AL: "Optimal maintenance treatment with calcipotriol/betamethasone dipropionate gel in Korean patients with psoriasis vulgaris: a multicentre randomized, controlled clinical trial", JEADV. JOURNAL OF THE EUROPEAN ACADEMY OF DERMATOLOGY AND VENEREOLOGY., vol. 31, no. 3, 10 October 2016 (2016-10-10), pages 483-489, XP055751625, NL ISSN: 0926-9959, DOI: 10.1111/jdv.13865
- DEL ROSSO DO JAMES Q: "Combination topical therapy for the treatment of psoriasis", JOURNAL OF DRUGS IN DERMATOLOGY, STRATEGIC COMMUNICATION IN DERMATOLOGY, NEW YORK, NY, US, vol. 5, no. 3, 1 March 2006 (2006-03-01), pages 232-234, XP009094031, ISSN: 1545-9616
- Anonymous: "Enstilar - Final Public Assessment Report - Scientific discussion", , 25 April 2017 (2017-04-25), pages 1-18, XP055752029, Retrieved from the Internet: URL:https://mri.cts-mrp.eu/human/downloads /DK_H_2478_001_PAR.pdf [retrieved on 2020-11-18]
- M LEBWOHL ET AL: "A phase 3 randomized, double-blind trial comparing the efficacy and safety of the fixed combination of calcipotriene 0.005% (Cal) and betamethasone dipropionate 0.064% (BD) aerosol foam with the aerosol foam vehicle used twice weekly as long-term maintenance", J. AM. ACAD. DERMATOL., vol. 76, no. 6, 1 June 2017 (2017-06-01), page AB21, XP055752032,
- SASCHA GERDES ET AL: "Prospective, Observational, Non-Interventional, Multicentre Study on the Efficacy and Tolerability of a New Calcipotriol/Betamethasone Aerosol Foam (Enstilar ) in Patients with Plaque Psoriasis under Daily Practice Conditions", DERMATOLOGY, vol. 233, no. 6, 1 January 2017 (2017-01-01), pages 425-434, XP055751620, CH ISSN: 1018-8665, DOI: 10.1159/000486700
- JILLIAN FRIEDER ET AL: "Calcipotriene betamethasone dipropionate aerosol foam in the treatment of plaque psoriasis: a review of the literature", THERAPEUTIC DELIVERY, vol. 8, no. 9, 1 August 2017 (2017-08-01), pages 737-746, XP055751616, GB ISSN: 2041-5990, DOI: 10.4155/tde-2017-0058
- LANATI EP ET AL: "Budget Impact Analysis of Enstilar for The Treatment of Psoriasis In Italy", VALUE IN HEALTH, vol. 20, no. 9, 1 October 2017 (2017-10-01), page A802, XP085221370, ISSN: 1098-3015, DOI: 10.1016/J.JVAL.2017.08.2387

## Description

### FIELD OF INVENTION

Psoriasis vulgaris is a chronic inflammatory condition, which is often treated with topical interventions, and is a life-long burden for many patients. Psoriasis is characterized by multiple flare ups and there is a real need for an effective and well tolerated, long-term topical treatment to reduce the number of flare ups.

The present invention relates to an anhydrous pharmaceutical composition comprising betamethasone dipropionate and calcipotriol for bi weekly administration for the prevention of flare ups and prolongation of flare-up free intervals in psoriasis patients that are in remission following treatment of their psoriasis

### BACKGROUND OF THE INVENTION

Psoriasis is a chronic inflammatory skin disease that manifests as erythematous, dry, scaling plaques resulting from hyperkeratosis. The plaques are most often found on the elbows, knees and scalp, though more extensive lesions may appear on other parts of the body, notably the lumbosacral region or scalp. A common treatment of mild to moderate psoriasis involves topical application of a composition containing a corticosteroid as the active ingredient. While efficacious, application of corticosteroids has the disadvantage of a number of adverse effects such as skin atrophy, striae, acneiform eruptions, perioral dermatitis, overgrowth of skin fungus and bacteria, hypopigmentation of pigmented skin and rosacea.

Psoriasis may also be treated with with antibodies, such as Adalimumab, Adalimumab-adbm, Brodalumab, Certolizumab pegol, Etanercept, Etanercept-szzs, Guselkumab, Infliximab, Ixekizumab, Risankizumab-rza, Secukinumab and Ustekinumab or oral formulations, such as formulations containing Apremilast.

A topical combination product for the treatment of psoriasis has been marketed by LEO Pharma under the trade name Daivobet^{®} ointment and gel. The product comprises calcipotriol monohydrate and betamethasone dipropionate as the active ingredients formulated in an composition comprising polyoxypropylene stearyl ether as a solvent.

A study on the treatment of psoriasis followed by a maintenance treatment using a gel comprising calcipotriol monhydrate and betamethasone dipropionate is disclosed in Lee JH et al; JEADV 2017, 31: 483-489 (XP055751625).

Another combination product for the treatment of psoriasis is marketed by LEO Pharma under the trade name Enstilar^{®} cutaneous foam. The product comprises calcipotriol monohydrate and betamethasone dipropionate as the active ingredients that is formulated in a white petrolatum based composition comprising polyoxypropylene stearyl ether wherein the actives are dissolved in the propellant.

It has been found that Enstilar^{®} cutaneous foam delivers more of the actives into the skin than Daivobet^{®} ointment and gel resulting in higher efficacy in the treatment of psoriasis, see Lind et al., Dermatol Ther (Heidelb) (2016) 6:413-25 and Queille-Roussel et al., Clin Drug Investig (2015) 35:239-45.

Enstillar^{®} cutaneous foam is an effective treatment for psoriasis vulgaris and is approved for once daily treatment of psoriasis vulgaris for up to four weeks, Summary of Product Characteristics for Enstillar^{®} cutaneous foam.

It has now has surprisingly been found that long term bi-weekly maintenance treatment with such foam compositions is safe in psoriasis patients.

It has also been found that the number of relapses in one year, the time to first relapse and the number of days in remission are considerably increased in patients treating themselves bi-weekly with such foam compositions when in remission compared to patients who do not treat themselves with such compositions when in remission. Accordingly, the present invention relates to foam compositions for use in maintenance therapy of psoriasis patients wherein said composition is administered bi-weekly, when patients are in remission.

### SUMMARY OF THE INVENTION

The present invention provides an anhydrous topical pharmaceutical composition comprising:
pharmaceutically acceptable lipid carrier comprising:
   about 0.005% w/w calcipotriol or about 0.00522 % w/w calcipotriol monohydrate,
   about 0.064 % w/w of betamethasone dipropionate, and petrolatum;
and in addition to said pharmaceutically acceptable lipid carrier a pharmaceutically acceptable propellant; for use in maintenance treatment of psoriasis patients, wherein when the psoriasis patient are in remission then said pharmaceutical composition is administered bi weekly, wherein bi-weekly means administration two times a week with 2 to 3 days between administrations, and wherein an improved therapeutic result is achieved compared to patients to whom said pharmaceutical composition is not administered when in remission.

According to the invention the improved therapeutic result is achieved using a lower cumulative amount of said pharmaceutical composition compared to patients to whom said pharmaceutical composition is only administered when the patient experiences a flare up of their psoriasis.

According to one embodiment of the invention the improved therapeutic result is a reduction in the number/rate of flare ups by up to 37-54 %.

According to one embodiment of the invention the improved therapeutic result is an increase the number of days until first relapse is increased by 80-90%.

According to one embodiment of the invention the improved therapeutic result is 5-15 % more days in remission per year.

According to one embodiment of the invention the improved therapeutic result is a reduction in the number of flare ups by up to 46 %.

According to one embodiment of the invention the improved therapeutic result is an increase in the number of days until first relapse by 87 %.

According to one embodiment of the invention the improved therapeutic result is 11 % more days in remission per year.

### DETAILED DESCRIPTION OF THE INVENTION

In a phase 3 randomized, double-blind, trial comparing the efficacy and safety of Composition G as described herein with placebo for bi-weekly maintenance treatment of psoriasis patients in remission has shown the following benefits:
Median time to first relapse was 56 days vs 30 days for Composition G and vehicle, respectively.
Risk of first relapse was reduced by 43% with Composition G vs vehicle (HR, 0.57; 95% CI, 0.47-0.69; P<0.0001).
Rate of relapse over one year was reduced by 46% (95% CI, 37-54%; P<0.001) for the Composition G group versus vehicle; predicted mean number of relapses over one year was 4.0 vs 7.5, Composition G and vehicle, respectively.
Patients in the Composition G group had 11% more days in remission than patients in the vehicle group (P<0.0001). Over one year this corresponds to 41 extra days in remission (95% CI, 29-51 days).
Composition G was well tolerated over the 52-week study period. Incidence of rebound was lower in the Composition G group compared with vehicle. There were no new cases of striae, cutaneous atrophy or clinically significant HPA axis suppression reported in either treatment group.

The present invention thus relates to the treatment of patients when in remission following treatment with one or more actives or compositions know to be effective in the treatment of psoriasis. Said actives or compositions may be any know active or composition useful for treating psoriasis such as Enstillar ^{®}, Daivobet^{®} gel or ointment, Daivonex cream or ointment, topical products containing corticosteroids or other topical products, antibodies such as Adalimumab, Adalimumab-adbm, Brodalumab, Certolizumab pegol, Etanercept, Etanercept-szzs, Guselkumab, Infliximab, Ixekizumab, Risankizumab-rza, Secukinumab and Ustekinumab, or oral formulations, such as formulations comprising Apremilast.

The composition for use according to the invention comprises pharmaceutically acceptable lipid carrier comprising
about 0.005% w/w calcipotriol or about 0.00522 % w/w calcipotriol monohydrate,
about 0.064 % w/w of betamethasone dipropionate,
and petrolatum;
and in addition to said pharmaceutically acceptable lipid carrier a pharmaceutically acceptable propellant.

Whenever reference is made to "said composition" herein reference is made to a composition comprising a pharmaceutically acceptable lipid carrier comprising
about 0.005% w/w calcipotriol or about 0.00522 % w/w calcipotriol monohydrate,
about 0.064 % w/w of betamethasone dipropionate,
and petrolatum;
and in addition to said pharmaceutically acceptable lipid carrier a pharmaceutically acceptable propellant.

As used herein in connection with an amount expressed in % w/w the term 'about' means plus/minus 10 % of that amount.

As used herein an anhydrous pharmaceutical composition is a composition to which water has not been added as an excipient or pharmaceutically inactive ingredient. The inactive ingredients added to the composition may contain small amounts of water as an impurity but none of the inactive ingredients contain any larger amount of water such as exceeding 1 % water.

Psoriasis is a chronic disease and psoriasis patients experience multiple flare ups of their disease every year.

As used herein patients in remission means subjects who have achieved treatment success after treatment with a medication effective for the treatment of psoriasis.

Flare up means an exacerbation of psoriasis symptoms, for example to a level defined as a PGA of at least 'mild'). Flare up and relapse have the same meaning as used herein.

PGA means Physicians Global Assessment of improvement, which is a recognized measure of psoriasis symptoms and which EMEA requires pharmaceutical companies to use clinical trials. PGA is assessed according to table 1 below

**Table 1 : Disease Severity Grading - Physician's Assessment**

| | |
|---|---|
| Clear (=0) | Plaque thickening = no elevation or thickening over normal skin |
| | Scaling = no evidence of scaling |
| | Erythema = none (no residual red colouration but post-inflammatory hyperpigmentation may be present) |
| Almost clear (= 1) | Plaque thickening = none or possible thickening but difficult to ascertain whether there is a slight elevation above normal skin level |
| | Scaling = none or residual surface dryness and scaling |
| | Erythema = light pink colouration |
| Mild (=2) | Plaque thickening = slight but definite elevation |
| | Scaling = fine scales partially or mostly covering lesions |
| | Erythema = light red colouration |
| Moderate (=3) | Plaque thickening = moderate elevation with rounded or sloped edges |
| | Scaling = most lesions at least partially covered |
| | Erythema = definite red colouration |
| Severe (=4) | Plaque thickening = marked elevation typically with hard or sharp edges |
| | Scaling = non-tenacious scale predominates, covering most or all of the lesions |
| | Erythema = very bright red colouration |

Maintenance treatment means a long term treatment that is carried out to prevent flare ups and prolong flare up free periods. As used herein maintenance treatment, long term maintenance treatment and proactive management have the same meaning.

As used herein bi-weekly means administration two times a week with 2 to 3 days between administrations.

The pharmaceutically acceptable lipid carrier for use according to the invention comprises the actives calcipotriol and betamethasone and petrolatum.

Petrolatum, white petrolatum or white soft paraffin is a frequently used lipid carrier which is composed of hydrocarbons of different chain lengths peaking at about C₄₀₋₄₄. White soft paraffin or whit petrolatum provides occlusion of the treated skin surface, reducing transdermal loss of water and potentiating the therapeutic effect of the active ingredient in the composition, it tends to have a greasy or tacky feel which persists for quite some time after application.

Liquid paraffin may be added to petrolatum to make it softer. Liquid paraffin consist of hydrocarbons of different chain lengths peaking at C₂₈₋₄₀.

It may be preferred to employ paraffins consisting of hydrocarbons of a somewhat lower chain length, such as paraffins consisting of hydrocarbons with chain lengths peaking at C₁₄₋₁₆, C₁₈₋₂₂, C₂₀₋₂₂, C₂₀₋₂₆ or mixtures thereof. It has been found that such paraffins are more cosmetically acceptable in that they are less greasy or tacky on application. The inclusion of such paraffins in the present composition is therefore expected to result in improved patient compliance. Suitable paraffins of this type, termed petrolatum jelly, are manufactured by Sonneborn and marketed under the trade name Sonnecone, e.g. Sonnecone CM, Sonnecone DM1, Sonnecone DM2 and Sonnecone HV. These paraffins are further disclosed and characterized in WO 2008/141078 .

In addition to their favourable cosmetic properties, it has surprisingly been found that compositions containing these paraffins as carriers are more tolerable than compositions containing conventional paraffins. (The hydrocarbon composition of the paraffins has been determined by gas chromatography).

The lipid carrier typically comprises of petrolatum in particular white petrolatum or white soft paraffin as the main component by weight and contains a liquid paraffin to soften the petrolatum.

Suitably the lipid carrier comprises more than 80 % w/w petrolatum, more than 85 % w/w petrolatum, or more than 90% w/w petrolatum and the petrolatum is suitably white soft paraffin or white petrolatum.

The composition used according to the invention may contain an anti-oxidant such as α-tocopherol or butyl hydroxy toluene.

The pharmaceutically acceptable propellant may be selected from dimethyl ether, methylethyl ether or C₃₋₅ alkanes, halogenated C₃₋₅ alkenes. Suitable C₃₋₅ alkanes are propane, n-butane, iso-butane or propane, pentane or mixtures thereof. A suitable halogenated C₃₋₅ alkenes is HFO-1234ze(E) (trans-1,3,3,3-tetrafluoroprop-1-ene).

In some embodiments the calcipotriol, calcipotriol monohydrate and/or betamethasone dipropionate is dissolved in the propellant.

According to one embodiment of the invention the pharmaceutically acceptable propellant comprises a C₃₋₅ alkane or halogenated C₃₋₅ alkene, such as n-butane.

In a specific embodiment of the invention the pharmaceutically acceptable propellant comprises dimethyl ether.

In another embodiment of the invention, the composition the pharmaceutically acceptable propellant is present in an amount sufficient to completely dissolve the calcipotriol or calcipotriol monohydrate and the betamethasone dipropionate.

According to one such an embodiment of the invention pharmaceutically acceptable propellant comprises dimethyl ether and the calcipotriol or calcipotriol monohydrate and the betamethasone dipropionate are dissolved in the dimethyl ether.

According to another such embodiment the pharmaceutically acceptable propellant comprises dimethyl ether and the calcipotriol or calcipotriol monohydrate and the betamethasone dipropionate are dissolved in the mixture of dimethyl ether and a second propellant. The second propellant is suitably C₃₋₅ alkane or a halogenated C₃₋₅ alkene, such as n-butane.

Suitably the ratio of the C₃₋₅ alkane or halogenated C₃₋₅ alkene to the dimethyl ether is in the range of about 6:1-0-5:1 w/w, about 5:1-1:2 w/w, about 4:1-1:1 w/w, about 4:2-1:1 w/w, 4:2-4:3 w/w or 4:3-1:1 w/w.

The lipid carrier in the formulation for use according to the invention is dissolved or suspended in the propellant.

The pharmaceutically acceptable lipid carrier may contain an oily co-solvent. The co-solvent may be selected from
(a) a compound of general formula I: H(OCH₂C(CH₃)H)ₓOR¹ wherein R¹ is a straight or branched chain C₁₋₂₀ alkyl, and x is an integer from 2 to 60 inclusive;
(b) an isopropyl ester of a straight or branched chain C₁₀₋₁₈ alkanoic or alkenoic acid;
(c) a propylene glycol diester of a C₈₋₁₄ alkanoic or alkenoic acid;
(d) a straight or branched C₈₋₂₄ alkanol or alkenol;
(e) a vegetable oil; and
(f) an N-alkylpyrrolidone or N-alkylpiperidone.

In one embodiment the pharmaceutically acceptable co-solvent is polyoxypropylene-15-stearyl ether, polyoxypropylene-11-stearyl ether, polyoxypropylene-14-butyl ether, polyoxypropylene-10-cetyl ether, or polyoxypropylene-3-myristyl ether.

In one embodiment the pharmaceutically acceptable co-solvent is isopropyl myristate, isopropyl palmitate, isopropyl isostearate, isopropyl linolate, or isopropyl monooleate.

In one embodiment the pharmaceutically acceptable co-solvent is propylene glycol dipelargonate.

In one embodiment the pharmaceutically acceptable co-solvent is capryl alcohol, lauryl alcohol, cetyl alcohol, stearyl alcohol, or myristyl alcohol, and the C₈₋₂₄ alkenol is oleyl alcohol or linoleyl alcohol, or wherein the branched C₈₋₂₄ alkanol is a branched C₁₈₋₂₄ alkanol.

In one embodiment the pharmaceutically acceptable co-solvent is myristyl alcohol or oleyl alcohol.

In one embodiment the pharmaceutically acceptable co-solvent is N-methylpyrrolidone.

In one embodiment the pharmaceutically acceptable co-solvent is a refined vegetable oil such as refined coconut oil or medium chain triglycerides.

In one embodiment the pharmaceutically acceptable co-solvent is polyoxypropylene-11-stearyl ether.

The amount of co-solvent used depend on the co-solvents ability to dissolve the actives ingredients but is typically in the range 0.5-25 % w/w, 0.5-10 % w/w, 1-5 % w/w or 3-5% w/w of the pharmaceutically acceptable lipid carrier. The amount of co-solvent is suitably below the amount sufficient to dissolve the actives in the formaulation.

In one embodiment the propellant is present in an amount that completely dissolve the actives and the amount of co-solvent is below the amount that is sufficient to dissolve the actives in the lipid carrier.

The topical composition used according to the of the invention is administered dermally or cutaneously. The composition is be sprayed onto skin areas affected by psoriasis flare ups, and then rubbed in by hand.

The compositions for use according to the invention may be prepared as described in WO2011154004 or by similar methods.

### Embodiments:

Embodiment 1: A method of maintenance treatment of psoriasis patients who are in remission wherein the method comprises treating patients bi weekly with a pharmaceutical composition comprising
   a pharmaceutically acceptable lipid carrier comprising:
   in addition to said pharmaceutically acceptable lipid carrier a pharmaceutically acceptable propellant;
      about 0.005% w/w calcipotriol or about 0.00522 % w/w calcipotriol monohydrate,
      about 0.064 % w/w of betamethasone dipropionate,
      and petrolatum, and
   wherein an improved therapeutic result is achieved compared to patients to whom said pharmaceutical composition is not administered when in remission.
Embodiment 2: A method for maintenance treatment of psoriasis patients who are in remission following up to up to 4 weeks of once daily treatment of flare ups with an anhydrous pharmaceutical composition comprising
   a pharmaceutically acceptable lipid carrier comprising:
      about 0.005% w/w calcipotriol or about 0.00522 % w/w calcipotriol monohydrate,
      about 0.064 % w/w of betamethasone dipropionate,
      and petrolatum, and
   in addition to said pharmaceutically acceptable lipid carrier a pharmaceutically acceptable propellant;
   wherein the method comprises treating the patients with said pharmaceutical composition bi weekly, and wherein an improved therapeutic result is achieved using a lower amount of said pharmaceutical composition compared to patients to whom said pharmaceutical composition is not administered when in remission.
Embodiment 3: The use of betamethasone dipropionate and calcipotriol or calcipotriol monohydrate in the manufacture of an anhydrous pharmaceutical composition comprising:
   a pharmaceutically acceptable lipid carrier comprising
      about 0.005% w/w calcipotriol or about 0.00522 % w/w calcipotriol monohydrate,
      about 0.064 % w/w of betamethasone dipropionate,
      and petrolatum, and
   in addition to said pharmaceutically acceptable lipid carrier a pharmaceutically acceptable propellant;
   for maintenance treatment of psoriasis patients who are in remission, wherein the anhydrous pharmaceutical composition is administered to the patients bi weekly, and wherein an improved therapeutic result is achieved compared to patients to whom said pharmaceutical composition is not administered when in remission.
Embodiment 4: use of betamethasone dipropionate and calcipotriol or calcipotriol monohydrate in the manufacture of a pharmaceutical composition for maintenance treatment of psoriasis patients who are in remission following up to 4 weeks of once daily treatment of flare ups with an anhydrous pharmaceutical composition comprising:
   a pharmaceutically acceptable lipid carrier comprising
      about 0.005% w/w calcipotriol or about 0.00522 % w/w calcipotriol monohydrate,
      about 0.064 % w/w of betamethasone dipropionate,
      and petrolatum, and
   in addition to said lipid carrier a pharmaceutically acceptable propellant;
   wherein the pharmaceutical composition is administered to the patients bi weekly, and wherein an improved therapeutic result is achieved using a lower amount of said composition compared to patients to whom said pharmaceutical composition is not administered when in remission.

### Example 1

### Compositions A-E and G

To prepare Compositions A-E and G white soft paraffin was melted at 80°C followed by cooling to 70°C and maintaining that temperature. Calcipotriol monohydrate was dissolved in polyoxypropylene-15-stearyl ether to form a solution which was added to the molten paraffin with stirring. BDP was dispersed in liquid paraffin and the dispersion was added to the caclipotriol-containing paraffin mixture with stirring, after which the mixture was cooled to below 30°C. 30 g portions of the mixture were transferred to aluminium spray containers provided with a polyamide-polyimide inner lacquer (HOBA 8460) after which a valve cup was fastened to the container body by crimping. The requisite amount of propellant mixture was added through a tube, after which the container was shaken for 5 minutes for complete dissolution of the calcipotriol and BDP.

### Composition A

| *Ingredients* | % *w*/*w* | % *w*/*w without the propellant* |
|---|---|---|
| Calcipotriol monohydrate | 0.002 | 0.005 |
| Betamethasone dipropionate | 0.026 | 0.064 |
| Liquid paraffin | 1.22 | 3 |
| α-tocopherol | 0.001 | 0.0025 |
| PPG-15-stearyl ether | 2.0 | 4.9 |
| White soft paraffin | 37.5 | 92 |

| | % of total composition | |
|---|---|---|
| Dimethyl ether | 31.7 | |
| Butane | 27.5 | |

### Composition B

| *Ingredients* | % *w*/*w* |
|---|---|
| Calcipotriol monohydrate | 0.002 |
| Betamethasone dipropionate | 0.02 |
| Liquid paraffin | 0.9 |
| α-tocopherol | 0.001 |
| PPG-15-stearyl ether | 1.6 |
| White soft paraffin | 28.9 |
| Dimethyl ether | 36.7 |
| Butane | 31.9 |

### Composition C

| *Ingredients* | % *w*/*w* |
|---|---|
| Calcipotriol monohydrate | 0.001 |
| Betamethasone dipropionate | 0.006 |
| Liquid paraffin | 0.3 |
| PPG-15-stearyl ether | 0.5 |
| White soft paraffin | 8.9 |
| Dimethyl ether | 90.3 |

### Composition D

| *Ingredients* | % *w*/*w* |
|---|---|
| Calcipotriol monohydrate | 0.002 |
| Betamethasone dipropionate | 0.030 |
| Liquid paraffin | 1.42 |
| α-tocopherol | 0.001 |
| PPG-15-stearyl ether | 2.4 |
| White soft paraffin | 43.6 |
| Dimethyl ether | 52.6 |

### Composition E

| *Ingredients* | *% w*/*w* | *% w*/*w without propellant* |
|---|---|---|
| Calcipotriol monohydrate | 0.002 | 0,005 |
| Betamethasone dipropionate | 0.026 | 0.064 |
| Liquid paraffin | 1.22 | 3 |
| α-tocopherol | 0.001 | 0.0025 |
| PPG-15-stearyl ether | 2.0 | 4.9 |
| White soft paraffin | 37.5 | 92 |

| | *% w*/*w of total composition* | |
|---|---|---|
| Dimethyl ether | 27.5 | |
| Butane | 31.7 | |

### Composition F

To prepare Composition F, hydrogenated castor oil is melted together with liquid paraffin at 85-90°C and cooled with homogenisation to about 60°C. The mixture is then cooled to 25-30°C with stirring. BDP is suspended in liquid paraffin and added to the homogenised mixture. Calcipotriol monohydrate is dissolved in polypropylene-15-stearyl ether and added to the mixture of the other ingredients, and the formulation was homogenised to ensure a homogenous distribution of the active ingredients. 30 g portions of the mixture are transferred to aluminium spray containers provided with a polyamide-polyimide inner lacquer (HOBA 8460) after which a valve cup is fastened to the container body by crimping. The requisite amount of propellant mixture is added through a tube, after which the container is shaken for 5 minutes for complete dissolution of the calcipotriol and BDP.

### Composition F

| *Ingredients* | *% w*/*w* | *% w*/*w without propellant* |
|---|---|---|
| Calcipotriol monohydrate | 0.002 | 0.005 |
| Betamethasone dipropionate | 0.03 | 0.073 |
| PPG-15-stearyl ether | 6.6 | 16 |
| Hydrogenated castor oil | 0.8 | 1.9 |
| Liquid paraffin | 33.6 | 82 |

| | | % w/w of total composition |
|---|---|---|
| Dimethyl ether | | 27.3 |
| Butane | | 31.7 |

### Composition G

| *Ingredients* | *% w*/*w* | *% w*/*w without propellant* |
|---|---|---|
| Calcipotriol monohydrate | 0.002 | 0,005 |
| Betamethasone dipropionate | 0.026 | 0.064 |
| Liquid paraffin | 1.22 | 3 |
| α-tocopherol | 0.001 | 0.0025 |
| PPG-11-stearyl ether(*) | 2.0 | 4.9 |
| White soft paraffin | 37.5 | 92 |

| | *% w*/*w of total composition* | |
|---|---|---|
| Dimethyl ether | 27.5 | |
| Butane | 31.7 | |

| | | |
|---|---|---|
| (*) PPG-1-stearyl ether comprise butyl hydroxy toluene as an antioxidant. | | |

### Composition H-N

To prepare Composition H-N, white soft paraffin and α-tocopherol are mixed by heating to approximately 80°C while stirring. The mixture is cooled to approximately 60°C. Micronized calcipotriol hydrate and BDP are dispersed in liquid paraffin and added to the molten paraffin while stirring. The mixture is then cooled to below 30°C while stirring. 30 g portions of the mixture are transferred to aluminium spray containers provided with a polyamide-polyimide inner lacquer (HOBA 8460) after which a valve cup is fastened to the container body by crimping. The requisite amount of propellant mixture is added through a tube, after which the container is shaken for 5 minutes for complete dissolution of the calcipotriol and BDP.

To prepare Compositions S-W, white soft paraffin and α-tocopherol are mixed by heating to approximately 80°C while stirring. The mixture is cooled to approximately 70°C. Calcipotriol monohydrate is dissolved in the co-solvents medium chain triglycerides, oleyl alcohol and isopropyl myristate respectively by heating to approximately 60°C to form a solution which is added to the molten paraffin α-tocopherol mixture while stirring. This mixture is then cooled to approximately 60°C and the temperature maintained. BDP is dispersed in liquid paraffin and the dispersion is added to the calcipotriol-containing paraffin mixture while stirring, after which the mixture is cooled to below 30°C while stirring. 30 g portions of the mixture are transferred to aluminium spray containers provided with a polyamide-polyimide inner lacquer (HOBA 8460) after which a valve cup is fastened to the container body by crimping. The requisite amount of propellant mixture is added through a tube, after which the container is shaken for 5 minutes for complete dissolution of the calcipotriol and BDP.

**Table 2: Formulations H-N without the weight contribution of the propellant contained 0.005 % w/w calcipotriol, 0.064 % w/w betamethasone dipropionate.**

| Ingredients (mg/g) | Aerosol compositions | | | | | | |
|---|---|---|---|---|---|---|---|
| | H | I | J | K | L | M | n |
| Calcipotriol | 0.02 | 0.02 | 0.02 | 0.02 | 0.02 | 0.02 | 0.02 |
| BDP | 0.251 | 0.263 | 0.251 | 0.263 | 0.251 | 0.263 | 0.263 |
| White soft paraffin | 378.6 | 396.7 | 359.1 | 376.2 | 374.7 | 392.6 | 376.2 |
| Liquid paraffin | 11.7 | 12.3 | 11.7 | 12.3 | 11.7 | 12.3 | 12.3 |
| Dimethyl ether | 609.4 | 274.2 | 609.4 | 274.2 | 609.4 | 274.2 | 274.2 |
| n-butane | 0.0 | 316.5 | 0.0 | 316.5 | 0.0 | 316.5 | 316.5 |
| Medium chain triglycerides | 0.0 | 0.0 | 19.5 | 20.5 | 0.0 | 0.0 | 0.0 |
| Oleyl alcohol | 0.0 | 0.0 | 0.0 | 0.0 | 3.9 | 4.1 | 0.0 |
| Isopropyl myristate | 0.0 | 0.0 | 0.0 | 0.0 | 0.0 | 0.0 | 20.5 |
| α-tocopherol | 0.008 | 0.008 | 0.008 | 0.008 | 0.008 | 0.008 | 0.008 |

### Example 2

### Clinical testing of efficacy

Composition G of example 1 was tested in a phase III, multi-centre, randomised, vehicle-controlled trial consisting of an open-label active-treatment phase (four weeks), a randomised, double-blinded proactive management phase (52 weeks) and an eight-week follow-up period.

After the screening phase, subjects deemed eligible for the trial (adults with psoriasis vulgaris on the trunk and/or limbs rated as at least 'mild' according to the PGA, BSA 2-30% and m-PASI of at least 2) was enrolled to the initial open-label phase, where they applied composition G once daily on the psoriatic lesions on trunk and/or limbs for up to 4 weeks.
- Subjects who achieve treatment success (PGA score of 'clear' or 'almost clear' with at least a 2-grade improvement from baseline) at Week 4 were randomised in a 1:1 ratio to a maintenance phase of 52 weeks with composition G or vehicle (maintenance IP) bi weekly.
- Subjects who do not achieve treatment success after up to 4 weeks of once daily treatment (i.e., non-responders) was discontinued from the trial.

During the randomised maintenance phase, subjects applied maintenance IP bi weekly 3 or 4 days apart (fixed days) on all areas on the trunk and limbs where lesions have cleared or almost cleared after the initial open-label phase or after relapse treatment. Subjects were assessed regularly at clinical visits (every up to 4 weeks). In addition, if in the opinion of the subject, a relapse (exacerbation of psoriasis) occurred between two regular monthly visits, the subject was assessed by the investigator at an unscheduled visit.

Following confirmation of a relapse the subject was provided with Composition G and asked to apply it to the active area(s) on trunk and/or limbs, irrespective of whether those areas were active at baseline or they are new lesions. Composition G was applied once daily on the active areas for up to 4 weeks. If additional areas became active during rescue treatment, these were treated once daily with Composition G. During a relapse, areas that are not active continued bi weekly maintenance treatment.
- If a score of 'clear'/'almost clear' according to the PGA was achieved after up to 4 weeks, the bi weekly maintenance regimen was re-started on the now 'clear'/'almost clear' area(s) according to the randomisation scheme.
- If a score of 'clear'/almost clear' according to the PGA was not achieved after the up to 4 weeks of once daily administration of Composition G, the subject left the trial.

Eligibility criteria: ≥18 years old; psoriasis vulgaris on trunk and/or limbs, involving 2-30% of body surface area; PGA at least 'mild' and modified psoriasis area and severity index score ≥2 at Visit 1.

Primary endpoint was time to first relapse (PGA at least 'mild'). Secondary endpoints: number of relapses and proportion of days in remission (PGA 'clear' or 'almost clear') during the proactive management phase. Safety was also assessed, including steroidrelated adverse events such as striae, cutaneous atrophy and hypothalamic pituitary adrenal (HPA) axis suppression (HPA axis testing was performed in a subset of patients at Week 0, 4, 28 and 56) (full results reported separately).

For each subject, the weight of medication used for each visit interval was be determined by calculating the difference between the weight of a set of full cans dispensed and the weight of the returned cans.

### Results

In total, 545 patients were randomised to receive composition G or vehicle, of whom 521 achieved treatment success in the open-label phase and comprised the full analysis set (Composition G) n=256; vehicle n=265); 251 (46.1%) of the 545 randomised patients completed the study. Disease characteristics at randomisation were similar across treatment groups. 82% of randomised patients had PGA score 'moderate' at baseline.

Median time to first relapse was 56 days vs 30 days for Composition G and vehicle, respectively.

Risk of first relapse was reduced by 43% with Composition G vs vehicle (HR, 0.57; 95% CI, 0.47-0.69; P<0.0001).

Rate of relapse over one year was reduced by 46% (95% CI, 37-54%; P<0.001) for the Composition G group versus vehicle; predicted mean number of relapses over one year was 4.0 vs 7.5, Composition G and vehicle, respectively.

Patients in the Composition G group had 11% more days in remission than patients in the vehicle group (P<0.0001). Over one year this corresponds to 41 extra days in remission (95% CI, 29-51 days). Composition G was well tolerated over the 52-week study period. Incidence of rebound was lower in the Composition G group compared with vehicle. There were no new cases of striae, cutaneous atrophy or clinically significant HPA axis suppression reported in either treatment group.

The long-term proactive management over 52 weeks with Composition G was superior in prolonging time to first relapse, reducing number of relapses and increasing days in remission versus vehicle in adults with psoriasis vulgaris.

In addition over a 52 weeks treatment period measured as g medication per day patients treated biweekly between relapses used 6 % by weight less of Composition G.

### Example 3

### Clinical testing safety

Patients achieving treatment success (physician's global assessment of disease severity [PGA] score 'clear'/'almost clear' with ≥2-grade improvement from baseline) following once-daily Composition G for up to 4 weeks, were randomized 1:1 to bi-weekly Composition G or vehicle for 52 weeks. Eligibility criteria: ≥18 years; truncal and/or limb psoriasis at least'mild' by PGA; involving 2-30% body surface area (BSA); modified psoriasis area and severity index score (mPASI) ≥2. Additional criteria for HPA axis subgroup: truncal and/or limb psoriasis at least'moderate' by PGA; involving 10-30% BSA; normal HPA axis function.

545 patients were randomized to Composition G (n=272) or vehicle (n=273). Characteristics at randomization were similar between groups. Rate of AEs per 100 patient-years was 165.1 and 156.1, Composition G and vehicle groups, respectively. Rate of serious AEs per 100 patient-years was low and comparable (8.2, Composition G; 7.8 vehicle), as was rate of treatment-related AEs (2.7, Composition G; 4.5, vehicle). Two AEs (chorioretinopathy and pain of skin) were adjudicated as related to long-term corticosteroid use. Three patients (2 Composition G [0.7%]; 1 vehicle [0.4%]) experienced AEs leading to discontinuation. Rebound within two months of entering the proactive-management phase occurred in six and seven patients, Composition G and vehicle, respectively. Rebound was four times as likely with vehicle (n=17) compared with Composition G (n=4) following relapse. No clinically relevant effect on calcium metabolism or HPA axis by subgroup analysis was observed.

HRQoL (Health Related Quality of Life) (was assessed using the EuroQol-5D for psoriasis (EQ-5D-5L-PSO) tool (0-1; 0=worst health state, 1=best health state) and the Dermatology Life Quality Index (DLQI) (0-30; 0=no impairment of life quality, 30=maximum impairment). Patient-perceived symptom severity was assessed using the Psoriasis Symptom Inventory (PSI) (0-32; 0=no severity, 32=high severity).

Statistically and clinically significant improvements were observed across all PRO (Patent-Reported Outcome) measures for flare treatment during the open-label phase. Specifically, the mean difference (standard deviation [SD]) from baseline to Week 4 was -8.97 (6.18) for PSI scores, -6.02 (5.46) for DLQI scores and 0.11 (0.15) for EQ-5D scores. The PRO improvements were maintained over the next 52 weeks of randomised treatment for both proactive and reactive management arms, across the three PRO assessment tools. After the initial improvement during the 4-week flare treatment, patients receiving reactive management had statistically significantly higher mean area under the curve (AUC) scores, both for DLQI (15% [p=0.007]) and PSI (Psoriasis Symptom Inventory) (15% [p=0.0128]), compared with patients receiving proactive management during the maintenance phase. Additionally, patients receiving reactive management also had a lower EQ-5D mean AUC score compared with patients receiving proactive management (1% [p=0.0842]) during the maintenance phase.

**Table 1: Changes in PSI, DLQI and EQ-5D scores in flare treatment from baseline to Week 4**

| | Baseline | | Week 4 | | Difference | | Statistical significance |
|---|---|---|---|---|---|---|---|
| | N | Mean(SD) | N | Mean(SD) | N | Mean(SD) | P-value |
| PSI | 471 | 12.5(6.15) | 360 | 3.36(3.66) | 330 | -8.97(6.18) | <0.0001 |
| DLQI | 519 | 8.63(6.19) | 516 | 2.64(3.31) | 515 | -6.02(5.46) | <0.0001 |
| EQ-5D | 518 | 0.80(0.17) | 515 | 0.90(0.14) | 513 | 0.11(0.15) | <0.0001 |

**Table 2: Mean PSI, DLQI and EQ-5D AUC scores in proactive and reactive arms and differences across during maintenance phase**

| | Proactive | Reactive | Difference | Statistical significance |
|---|---|---|---|---|
| PSI | 4.99 | 5.74 | -0.75 | P=0.0128 |
| DLQI | 2.95 | 3.40 | -0.45 | P=0.007 |
| EQ-5D | 0.89 | 0.88 | 0.01 | P=0.0842 |

## Claims

1. An anhydrous topical pharmaceutical composition comprising
pharmaceutically acceptable lipid carrier comprising:
about 0.005% w/w calcipotriol or about 0.00522 % w/w calcipotriol monohydrate,
about 0.064 % w/w of betamethasone dipropionate, and
petrolatum;
and in addition to said pharmaceutically acceptable lipid carrier a pharmaceutically acceptable propellant;
for use in maintenance treatment of psoriasis patients,
wherein when the psoriasis patient are in remission then said pharmaceutical composition is administered bi weekly, wherein bi-weekly means administration two times a week with 2 to 3 days between administration, and
wherein an improved therapeutic result is achieved compared to patients to whom said pharmaceutical composition is not administered when in remission.

2. The composition for use according to claim 1, wherein the improved therapeutic result is a reduction in the number of flare ups by up to 37-54 %.

3. The composition for use according to any one of claims 1-2, wherein the improved therapeutic result is an increase in the number of days until relapse by 80-90%.

4. The composition for use according to any one of claims 1-3, wherein the improved therapeutic result is 5-15 % more days more days in remission.

5. The composition for use according to claim 1-4, wherein the pharmaceutically acceptable lipid carrier further comprises liquid paraffin.

6. The composition for use according to any one of claims 1-5, wherein the composition further comprises an oily co-solvent.

7. The composition for use according to claim 6, wherein the oily co-solvent comprises at least one of
(a) a compound of general formula I: H(OCH₂C(CH₃)H)ₓOR¹ wherein R¹ is a straight or branched chain C₁₋₂₀ alkyl, and x is an integer from 2 to 60 inclusive;
(b) an isopropyl ester of a straight or branched chain C₁₀₋₁₈ alkanoic or alkenoic acid;
(c) a propylene glycol diester of a C₈₋₁₄ alkanoic or alkenoic acid;
(d) a straight or branched C₈₋₂₄ alkanol or alkenol;
(e) a vegetable oil; and
(f) an N-alkylpyrrolidone or N-alkylpiperidone.

8. The composition for use according to claim 7, wherein the N-alkylpyrrolidone is N-methylpyrrolidone.

9. The composition for use according to claim 7, wherein the vegetable oil is medium chain triglycerides.

10. The composition for use according to claim 7, wherein the oily co-solvent comprises polyoxypropylene-11-stearyl ether.

11. The composition for use according to anyone of claims 1-10 where the propellant is 30-80 % w/w, 40-70 % w/w or 55 - 65 % w/w of the total composition.

12. The composition for use according to any of claims 1-11, wherein the pharmaceutically acceptable propellant comprises a C₃₋₅ alkane.

13. The composition for use according to any of claims 1-12, wherein the pharmaceutically acceptable propellant is present in an amount sufficient to dissolve the calcipotriol or calcipotriol monohydrate and/or the betamethasone dipropionate.

14. The composition for use according to any one of claims 1-13, wherein the pharmaceutically acceptable propellant comprises dimethyl ether.

15. The composition for use according to claim 1, wherein the pharmaceutically acceptable lipid carrier comprises:
75-95 % w/w white soft paraffin,
0.5 -10 % w/w liquid paraffin and
0 to 10 % w/w of a co-solvent;
and
the propellant is present in an amount corresponding to 30-80 % w/w of the total composition.

16. The composition for use according to claim 1, wherein,
the pharmaceutically acceptable lipid carrier comprises:
85-95 % w/w white soft paraffin,
0.5-10 % w/w liquid paraffin and
0 to 10 % w/w of a co-solvent;
and
the propellant is present in an amount corresponding to 40-70 % w/w of the total composition.

17. The composition for use according to claim 1, wherein,
The pharmaceutically acceptable lipid carrier comprising:
85-95 % w/w white soft paraffin,
0.5 -10 % w/w liquid paraffin and
0.5 to 10 % w/w of a co-solvent;
and
the propellant is present in an amount corresponding to 55-65 % w/w of the total composition.

18. The composition for use according to claim 1 wherein,
the pharmaceutically acceptable lipid carrier comprises:
85-95 % w/w white soft paraffin,
1-3 % w/w liquid paraffin and
3-7 % w/w of a co-solvent;
and
the propellant is present in an amount corresponding to 55-65 % w/w of the total composition.

19. The compositions for use according to claim 1 wherein,
the pharmaceutically acceptable lipid carrier comprises:
92 % w/w white soft paraffin,
2 % w/w liquid paraffin and
5 % w/w polyoxypropylene-11-stearylether;
and
the propellant is present in an amount corresponding to 58-62 % w/w of the total composition.

20. The composition for use according to any one of claims 15-19 wherein the propellant comprises dimethylether.

21. The composition for use according to any one of claims 15-20 wherein the propellant comprise butane or a mixture of butane and propane.

22. The composition for use according to one of any of claims 1-21 comprising an antioxidant.

## Patentansprüche

1. Wasserfreie topische pharmazeutische Zusammensetzung, umfassend pharmazeutisch verträglichen Lipid-Träger, umfassend:
etwa 0,005 % w/w Calcipotriol oder etwa 0,00522 % w/w Calcipotriolmonohydrat,
etwa 0,064 % w/w Betamethasondipropionat und
Vaseline;
und zusätzlich zu dem pharmazeutisch verträglichen Lipid-Träger ein pharmazeutisch verträgliches Treibmittel;
zur Verwendung bei der Erhaltungsbehandlung von Psoriasis-Patienten,
wobei, wenn sich die Psoriasis-Patienten in Remission befinden, die pharmazeutische Zusammensetzung zweimal wöchentlich verabreicht wird, wobei zweimal wöchentlich eine Verabreichung zweimal pro Woche mit 2 bis 3 Tagen zwischen der Verabreichung bedeutet, und
wobei ein verbessertes therapeutisches Ergebnis im Vergleich zu Patienten erzielt wird, denen die pharmazeutische Zusammensetzung nicht verabreicht wird, wenn sie sich in Remission befinden.

2. Zusammensetzung zur Verwendung nach Anspruch 1, wobei das verbesserte therapeutische Ergebnis eine Reduktion der Anzahl von Ausbrüchen um bis zu 37-54 % ist.

3. Zusammensetzung zur Verwendung nach einem der Ansprüche 1-2, wobei das verbesserte therapeutische Ergebnis eine Erhöhung der Anzahl von Tagen bis zum Rezidiv um 80-90 % ist.

4. Zusammensetzung zur Verwendung nach einem der Ansprüche 1-3, wobei das verbesserte therapeutische Ergebnis 5-15 % mehr Tage in Remission ist.

5. Zusammensetzung zur Verwendung nach Anspruch 1-4, wobei der pharmazeutisch verträgliche Lipid-Träger ferner flüssiges Paraffin umfasst.

6. Zusammensetzung zur Verwendung nach einem der Ansprüche 1-5, wobei die Zusammensetzung ferner ein öliges Co-Lösungsmittel umfasst.

7. Zusammensetzung zur Verwendung nach Anspruch 6, wobei das ölige Co-Lösungsmittel mindestens eines von Folgendem umfasst:
(a) einer Verbindung der allgemeinen Formel I: H(OCH₂C(CH₃)H)ₓOR¹, wobei R¹ ein gerad- oder verzweigtkettiges C₁₋₂₀-Alkyl ist und x eine ganze Zahl von 2 bis einschließlich 60 ist;
(b) einem Isopropylester einer gerad- oder verzweigtkettigen C₁₀₋₁₈-Alkan- oder -Alkensäure;
(c) einem Propylenglycoldiester einer C₈₋₁₄-Alkan- oder - Alkensäure;
(d) einem geraden oder verzweigten C₈₋₂₄-Alkanol oder - Alkenol;
(e) einem Pflanzenöl; und
(f) einem N-Alkylpyrrolidon oder N-Alkylpiperidon.

8. Zusammensetzung zur Verwendung nach Anspruch 7, wobei das N-Alkylpyrrolidon N-Methylpyrrolidon ist.

9. Zusammensetzung zur Verwendung nach Anspruch 7, wobei es sich bei dem Pflanzenöl um mittelkettige Triglyceride handelt.

10. Zusammensetzung zur Verwendung nach Anspruch 7, wobei das ölige Co-Lösungsmittel Polyoxypropylen-11-stearylether umfasst.

11. Zusammensetzung zur Verwendung nach einem der Ansprüche 1-10, wobei das Treibmittel 30-80 % w/w, 40-70 % w/w oder 55-65 % w/w der gesamten Zusammensetzung beträgt.

12. Zusammensetzung zur Verwendung nach einem der Ansprüche 1-11, wobei das pharmazeutisch verträgliche Treibmittel ein C₃₋₅-Alkan umfasst.

13. Zusammensetzung zur Verwendung nach einem der Ansprüche 1-12, wobei das pharmazeutisch verträgliche Treibmittel in einer Menge vorhanden ist, die ausreichend ist, um das Calcipotriol oder Calcipotriolmonohydrat und/oder das Betamethasondipropionat aufzulösen.

14. Zusammensetzung zur Verwendung nach einem der Ansprüche 1-13, wobei das pharmazeutisch verträgliche Treibmittel Dimethylether umfasst.

15. Zusammensetzung zur Verwendung nach Anspruch 1, wobei der pharmazeutisch verträgliche Lipid-Träger Folgendes umfasst:
75-95 % w/w weißes weiches Paraffin,
0,5-10 % w/w flüssiges Paraffin und
0 bis 10 % w/w eines Co-Lösungsmittels;
und
das Treibmittel in einer Menge vorhanden ist, die 30-80 % w/w der gesamten Zusammensetzung entspricht.

16. Zusammensetzung zur Verwendung nach Anspruch 1, wobei der pharmazeutisch verträgliche Lipid-Träger Folgendes umfasst:
85-95 % w/w weißes weiches Paraffin,
0,5-10 % w/w flüssiges Paraffin und
0 bis 10 % w/w eines Co-Lösungsmittels;
und
das Treibmittel in einer Menge vorhanden ist, die 40-70 % w/w der gesamten Zusammensetzung entspricht.

17. Zusammensetzung zur Verwendung nach Anspruch 1, wobei der pharmazeutisch verträgliche Lipid-Träger Folgendes umfasst: 85-95 % w/w weißes weiches Paraffin,
0,5-10 % w/w flüssiges Paraffin und
0,5 bis 10 % w/w eines Co-Lösungsmittels;
und
das Treibmittel in einer Menge vorhanden ist, die 55-65 % w/w der gesamten Zusammensetzung entspricht.

18. Zusammensetzung zur Verwendung nach Anspruch 1, wobei der pharmazeutisch verträgliche Lipid-Träger Folgendes umfasst:
85-95 % w/w weißes weiches Paraffin,
1-3 % w/w flüssiges Paraffin und
3-7 % w/w eines Co-Lösungsmittels;
und
das Treibmittel in einer Menge vorhanden ist, die 55-65 % w/w der gesamten Zusammensetzung entspricht.

19. Zusammensetzungen zur Verwendung nach Anspruch 1, wobei der pharmazeutisch verträgliche Lipid-Träger Folgendes umfasst:
92 % w/w weißes weiches Paraffin,
2 % w/w flüssiges Paraffin und
5 % w/w Polyoxypropylen-11-stearylether;
und
das Treibmittel in einer Menge vorhanden ist, die 58-62 % w/w der gesamten Zusammensetzung entspricht.

20. Zusammensetzung zur Verwendung nach einem der Ansprüche 15-19, wobei das Treibmittel Dimethylether umfasst.

21. Zusammensetzung zur Verwendung nach einem der Ansprüche 15-20, wobei das Treibmittel Butan oder ein Gemisch aus Butan und Propan umfasst.

22. Zusammensetzung zur Verwendung nach einem der Ansprüche 1-21, die ein Antioxidans umfasst.

## Revendications

1. Composition pharmaceutique topique anhydre comprenant un support lipidique pharmaceutiquement acceptable comprenant :
environ 0,005 % p/p de calcipotriol ou environ 0,00522 % p/p de monohydrate de calcipotriol,
environ 0,064 % p/p de dipropionate de bétaméthasone, et
de la vaseline ;
et en plus dudit support lipidique pharmaceutiquement acceptable, un propulseur pharmaceutiquement acceptable ;
destinée à être utilisée dans le traitement de maintien des patients atteints de psoriasis,
dans laquelle, lorsque le patient atteint de psoriasis est en rémission, alors ladite composition pharmaceutique est administrée de manière bihebdomadaire, dans laquelle de manière bihebdomadaire signifie une administration deux fois par semaine avec 2 à 3 jours entre les administrations, et
dans laquelle un résultat thérapeutique amélioré est observé par rapport à des patients auxquels ladite composition pharmaceutique n'est pas administrée lorsqu'ils sont en rémission.

2. Composition destinée à être utilisée selon la revendication 1, dans laquelle le résultat thérapeutique amélioré est une réduction du nombre de poussées allant jusqu'à 37 à 54 %.

3. Composition destinée à être utilisée selon l'une quelconque des revendications 1 et 2, dans laquelle le résultat thérapeutique amélioré est une augmentation du nombre de jours jusqu'à la rechute de 80 à 90 %.

4. Composition destinée à être utilisée selon l'une quelconque des revendications 1 à 3, dans laquelle le résultat thérapeutique amélioré est de 5 à 15 % de jours supplémentaires en rémission.

5. Composition destinée à être utilisée selon les revendications 1 à 4, dans laquelle le support lipidique pharmaceutiquement acceptable comprend en outre de la paraffine liquide.

6. Composition destinée à être utilisée selon l'une quelconque des revendications 1 à 5, dans laquelle la composition comprend en outre un co-solvant huileux.

7. Composition destinée à être utilisée selon la revendication 6, dans laquelle le co-solvant huileux comprend au moins l'un parmi
(a) un composé de formule générale I : H(OCH₂C(CH₃)H)ₓOR¹ dans lequelle R¹ est un alkyle en C₁₋₂₀ à chaîne droite ou ramifiée, et x est un nombre entier de 2 à 60, bornes comprises ;
(b) un ester isopropylique d'un acide alcanoïque ou alcénoïque en C₁₀₋₁₈ à chaîne droite ou ramifiée ;
(c) un diester de propylène glycol d'un acide alcanoïque ou alcénoïque en C₈₋₁₄ ;
(d) un alcanol ou un alcénol en C₈₋₂₄ linéaire ou ramifié ;
(e) une huile végétale ; et
(f) une N-alkylpyrrolidone ou une N-alkylpipéridone.

8. Composition destinée à être utilisée selon la revendication 7, dans laquelle la N-alkylpyrrolidone est la N-méthylpyrrolidone.

9. Composition destinée à être utilisée selon la revendication 7, dans laquelle l'huile végétale est constituée de triglycérides à chaîne moyenne.

10. Composition destinée à être utilisée selon la revendication 7, dans laquelle le co-solvant huileux comprend du polyoxypropylène-11-stéaryléther.

11. Composition destinée à être utilisée selon l'une quelconque des revendications 1 à 10, dans laquelle le propulseur représente 30 à 80 % p/p, 40 à 70 % p/p ou 55 à 65 % p/p de la composition totale.

12. Composition destinée à être utilisée selon l'une quelconque des revendications 1 à 11, dans laquelle le propulseur pharmaceutiquement acceptable comprend un alcane en C₃₋₅.

13. Composition destinée à être utilisée selon l'une quelconque des revendications 1 à 12, dans laquelle le propulseur pharmaceutiquement acceptable est présent en une quantité suffisante pour dissoudre le calcipotriol ou le monohydrate de calcipotriol et/ou le dipropionate de bétaméthasone.

14. Composition destinée à être utilisée selon l'une quelconque des revendications 1 à 13, dans laquelle le propulseur pharmaceutiquement acceptable comprend du diméthyléther.

15. Composition destinée à être utilisée selon la revendication 1, dans laquelle le support lipidique pharmaceutiquement acceptable comprend :
75 à 95 % p/p de paraffine molle blanche,
0,5 à 10 % p/p de paraffine liquide et
0 à 10 % p/p d'un co-solvant ;
et
le propulseur est présent en une quantité correspondant à 30 à 80 % p/p de la composition totale.

16. Composition destinée à être utilisée selon la revendication 1, dans laquelle le support lipidique pharmaceutiquement acceptable comprend :
85 à 95 % p/p de paraffine molle blanche,
0,5 à 10 % p/p de paraffine liquide et
0 à 10 % p/p d'un co-solvant ;
et
le propulseur est présent en une quantité correspondant à 40 à 70 % p/p de la composition totale.

17. Composition destinée à être utilisée selon la revendication 1, dans laquelle le support lipidique pharmaceutiquement acceptable comprend :
85 à 95 % p/p de paraffine molle blanche,
0,5 à 10 % p/p de paraffine liquide et
0,5 à 10 % p/p d'un co-solvant ;
et
le propulseur est présent en une quantité correspondant à 55 à 65 % p/p de la composition totale.

18. Composition destinée à être utilisée selon la revendication 1, dans laquelle le support lipidique pharmaceutiquement acceptable comprend :
85 à 95 % p/p de paraffine molle blanche,
1 à 3 % p/p de paraffine liquide et
3 à 7 % p/p d'un co-solvant ;
et
le propulseur est présent en une quantité correspondant à 55 à 65 % p/p de la composition totale.

19. Composition destinée à être utilisée selon la revendication 1, dans laquelle le support lipidique pharmaceutiquement acceptable comprend :
92 % p/p de paraffine molle blanche,
2 % p/p de paraffine liquide et
5 % p/p de polyoxypropylène-11-stéaryléther ;
et
le propulseur est présent en une quantité correspondant à 58 à 62 % p/p de la composition totale.

20. Composition destinée à être utilisée selon l'une quelconque des revendications 15 à 19, dans laquelle le propulseur comprend du diméthyléther.

21. Composition destinée à être utilisée selon l'une quelconque des revendications 15 à 20, dans laquelle le propulseur comprend du butane ou un mélange de butane et de propane.

22. Composition destinée à être utilisée selon l'une quelconque des revendications 1 à 21, comprenant un antioxydant.
